# EUROPEAN PATENT APPLICATION

(11) **EP 4 671 267 A1**
(43) Date of publication of application: **31.12.2025**
(21) Application number: 25184874.3
(22) Date of filing: 24.06.2025
(51) Int. Cl.: C07K 14/415, C12N 15/82

(54) **PROMOTER FOR REGULATING PLANT GENE EXPRESSION AND USES THEREOF**

(30) Priority: 27.06.2024 PL 44901824
(71) Applicant: Instytut Genetyki Roslin Polskiej Akademii Nauk, 60-479 Poznan (PL)
(72) Inventor: GREGORY, Franklin, 60-479 Poznan (PL); SINHA, Rakesh Kumar, 62-023 Gadki (PL)
(74) Representative: AOMB Polska Sp. z.o.o.

(57) **Abstract**

The invention relates to a promoter for regulating plant gene expression. Preferably, the promoter originates from a plant of the *Hypericaceae* family, specifically isolated from *Hypericum perforatum* (HyPRO promoter). The promoter sequence is activated in response to biotic and abiotic stresses and has been effectively utilized in recombinant genetic constructs, resulting in enhanced gene expression across various biological systems. The invention encompasses methods of isolation, characterization and application of the promoters according to the invention in genetic engineering, particularly in the development of stress-resistant and growth-promoting plants, opening up new possibilities in the field of plant biotechnology, enabling the development of novel crop cultivation strategies in changing environmental conditions.

## Description

### Field of the invention

The present invention relates to a promoter for regulating plant gene expression. Preferably, the promoter originates from a plant of the *Hypericaceae* family, specifically isolated from *Hypericum perforatum* (and accordingly is herein below referred to as HyPRO promoter). The promoter exhibits inducible properties, activating in response to biotic and/or abiotic stresses. The invention also relates to a recombinant cassette, a host plant cell transformed with a vector containing the recombinant cassette, a plant, its part, propagule or progeny thereof expressing the recombinant cassette, or a transformed plant cell, use of the promoter for the enhanced expression of a nucleotide sequence in an organism, a method of introducing a vector containing the recombinant cassette for modifying gene expression in a plant, preferably *Nicotiana tabacum,* a method for producing a plant with modified gene expression, and a method for obtaining an inducible promoter.

The promoter according to the invention has been effectively utilized in recombinant constructs, showing increased gene expression when coupled with target gene sequences and termination signals. The invention encompasses methods of isolation, characterization and application of the promoters according to the invention in genetic engineering, particularly in the development of stress-resistant and growth-promoting plants.

### Background art

A promoter is a crucial DNA sequence that facilitates the binding of RNA polymerases and initiates the process of transcription or replication. Consisting of a start site and additional regulatory elements, these sequences collectively enable polymerase binding and the initiation of gene transcription or DNA replication. Promoters are classified into constitutive types, which are active in all cells at all times, and specific types, including tissue-specific and inducible ones. The latter are activated in response to various factors such as biotic and abiotic stresses. Current research in biotechnology is focused on developing inducible promoters that can be flexibly activated in response to different stresses, thereby minimizing their negative impacts on plant development and the environment. Such innovations are crucial for breeding plants resistant to adverse conditions and advancing biotechnological applications.

CN106967720B relates to the cloning and application of the SlWRKY31P promoter, which is activated by various stresses such as high salinity, drought, or pathogenic bacterial invasion. This promoter, derived from tomato (*Solanum Lycopersicon*), is characterized by its nucleotide sequence consisting of 1849 nucleotides. The aim is to enhance the tolerance and resistance of transgenic plants to these environmental challenges by ensuring high expression of target genes under adverse conditions. The invention includes the development of an expression cassette containing this promoter and a recombinant vector, named pBI121SX-SlWRKY31P. This vector is designed for transformation into *Escherichia coli or Agrobacterium tumefaciens EHA105,* and subsequently into transgenic plants. The document also provides a detailed description of the primer pairs used for amplifying the SlWRKY31P promoter, the PCR methods for its extraction and amplification, and the methods used to study the promoter's induced expression in plants. Additionally, the GUS gene is employed as a reporter gene under the control of the SlWRKY31P promoter to monitor its activity.

CN107236731B describes the development of a promoter named PRJA140, which is activated by methyl jasmonate and bacterial blight. This promoter allows for precise regulation of exogenous resistance gene expression in plants, making it highly useful in plant genetic engineering for adjusting gene expression in response to environmental stresses or pathogen attacks. The production process for this promoter starts with the extraction of genomic DNA from rice (*Oryza sativa*). Specific primers are then used for PCR amplification to obtain the desired DNA sequence, which is subsequently validated and sequenced. The PRJA140 promoter is then inserted into a recombinant expression vector containing the β-glucuronidase (GUS) reporter gene, which is used to assess promoter activity. The vector, based on the pCAMBIA1381 backbone, highlights the key advantage of the PRJA140 promoter: its inducibility by both methyl jasmonate - a plant hormone linked to stress responses and pest defence-and bacterial blight. Normally, the promoter shows low activity, as evidenced by minimal expression of the GUS reporter gene. However, upon induction by either methyl jasmonate or bacterial blight, there is a significant increase in GUS gene expression, demonstrating the promoter's high reactivity and efficiency in activating target gene expression.

Further, US20200299708A1 relates to a nucleic acid vector designed for plant transformation. This vector features a promoter functionally linked to a heterologous polynucleotide sequence, which may include a polylinker sequence, a sequence encoding a PR1 protein not found in *Zea Mays* (corn), or both. The promoter shares at least 95% sequence identity with a specified reference sequence and spans 2000 base pairs. Intended uses of these vectors include transforming a variety of plants to install specific traits such as insect resistance, herbicide tolerance, and improved nitrogen or water use efficiency, as well as enhancing the nutritional value or other desirable characteristics of transgenic plants. Plants that can be transformed using this vector include, but are not limited to, *Zea Mays* (corn), wheat, rice, sorghum, oats, rye, bananas, sugarcane, cotton, Arabidopsis, tobacco, sunflower, and rapeseed.

Yet further, CN105543226B focuses on the isolation and application of the anthocyanin synthase promoter from the tea plant (CsANS). This work addresses the limitations of existing methods by providing promoters that are easy to use and yield stable, reliable results in regulating anthocyanin synthesis in plants. The process begins by isolating the CsANS promoter from the tea plant. The detailed sequence of this promoter, identified as SEQ ID NO: 1, is located upstream of the CsANS gene. Subsequently, recombinant vectors are created, and a thorough regulatory analysis of the promoter is performed.

### Summary of the invention

Given the limitations of the current state of the art, the aim of this invention is to provide promoters that facilitate easy handling and deliver stable and reliable results in gene expression regulation, particularly in response to biotic and/or abiotic stresses.

Accordingly, in its first aspect the present invention relates to a promoter comprising a sequence selected from:
(a) a nucleic acid sequence defined in SEQ ID NO: 1, or
(b) a functional portion thereof, or
(c) a functional derivative or a homologous promoter which is at least 70% similar to one of them.

Preferably, promoter is inducible. Particularly preferably, promoter is activated in response to a biotic and/or abiotic stress.

Preferably, biotic stress includes any plant pathogen or beneficial microorganism. Preferably, abiotic stress is caused by at least one stressor selected from nanoparticles, excessive or insufficient light, high or low temperature, drought, hormones, and chemicals.

Preferably, promoter is derived from the plant kingdom. Preferably, said sequence is derived from the *Hypericaceae family.* Preferably, said plant belonging to the genus *Hypericum.* Particularly preferably, said sequence is derived from *Hypericum perforatum.*

In a second aspect the present invention relates to a recombinant cassette comprising: (i) the nucleic acid sequence defined in SEQ ID NO: 1 operably linked to (ii) a polynucleotide sequence encoding a gene product to be transcribed from the inducible promoter, and (iii) a termination sequence.

In a third aspect the present invention relates to a host plant cell transformed with a vector containing the recombinant cassette defined above.

In a fourth aspect the present invention relates to a plant, its part, propagule or progeny thereof expressing the recombinant cassette defined above, or a transformed plant cell defined above.

Preferably, the plant belongs to the *Dicotyledons,* and most preferably is *Nicotiana tabacum.*

In a fifth aspect the present invention relates to use of the promoter defined above for enhancing the expression of a nucleotide sequence in an organism.

Preferably, in the use defined above the nucleotide sequence is a gene. Preferably, in the use defined above the organism is a eukaryote, more preferably belonging to the plant kingdom. Particularly preferably, in the use defined above the gene confers resistance of the plant to biotic and/or abiotic stress factors or promotes plant growth.

In a sixth aspect the present invention relates to a method of introducing a vector containing the recombinant cassette defined above for modifying gene expression in a plant, preferably *Nicotiana tabacum.*

In a seventh aspect the present invention relates to a method for producing a plant with modified gene expression, comprising the following steps:
(a) transforming a plant cell, tissue, organ or embryo with a vector containing the recombinant cassette defined above;
(b) selecting the transformed cells, cell calli, embryos or seeds;
(c) regenerating plants from transformed cells, cell calli, embryos or seeds selected in step (b);
(d) selecting mature plants from step (c) which have modified gene expression compared to non-transformed plants.

In an eighth aspect the present invention relates to a method for obtaining an inducible promoter as defined above, which is activated in response to a biotic and/or abiotic stress, the method, comprising the following steps:
(a) isolating genomic DNA from leaves of *Hypericum perforatum*;
(b) identifying the promoter sequence;
(c) cloning the identified promoter into suitable expression vectors using PCR cloning techniques;
(d) ligation of the DNA into a cloning vector;
(e) selection of positive colonies;
(f) sequencing the cloned plasmids to confirm the correctness of the promoter sequence.

Preferably, the step (c) comprises PCR amplification using the forward primer having the nucleic acid sequence defined in SEQ ID NO: 2 and the reverse primer having the nucleic acid sequence defined in SEQ ID NO: 3 to obtain a PCR amplification product.

In particular, the present invention introduces a HyPRO promoter, derived from the Hypericaceae family, and more specifically *Hypericum perforatum.* This promoter, consisting of 1116 base pairs, is activated by various stresses including plant pathogens, microorganisms, nanoparticles, temperature, drought, hormones, and chemicals. The invention encompasses also a recombinant cassette featuring this promoter, alongside a polynucleotide encoding a gene product and a termination sequence. Additionally, it includes a vector housing the cassette, a host plant cell transformed with this vector, and a transgenic plant expressing the cassette. The promoter's primary use is to enhance the expression of nucleotide sequences in plants, thereby conferring resistance to biotic and/or abiotic stresses or promoting plant growth. Methods for introducing this vector into plants and producing plants with modified gene expression are also part of the invention.

### Brief description of the figures of drawing

The invention will be further elucidated in the following non-limiting exemplary embodiments, with reference to the accompanying figures, wherein:
Fig. 1 shows a nucleotide sequence SEQ ID NO: 1 of the HyPRO promoter according to the invention with cis-acting regulatory motifs;
Fig. 2 shows a schematic map of the T-DNA region of the binary vector containing GUS gene under the control of HyPRO promoter according to the invention;
Fig. 3 shows images of Petri dishes with wild-type seedlings with no GUS expression, seedlings under the HyPRO promoter, and seedlings under the CaMV35S promoter (upper row, from left to right) and of close-ups of the same three seedings after GUS staining (lower row, left to right, in the same order);
Fig. 4 shows images of shoots, roots, root tips and flower buds of transgenic plants of *Nicotiana tabacum* having HyPRO full length, truncated and positive control having CaMV35S promoter linked to GUS, obtained in GUS transient assays;
Fig. 5 shows images obtained in GUS analysis of transgenic seedlings after treatment with hydrogen peroxide (H₂O₂);
Fig. 6 shows images obtained in GUS analysis of transgenic seedlings after treatment with abiotic stress inducers namely polyethylene glycol (PEG), salicylic acid (SA), Nitric oxide (NO) and cold;
Fig. 7 shows images obtained in GUS analysis of transgenic seedlings after treatment with *Agrobacterium tumefaciens;*
Fig. 8 shows images obtained in GUS analysis of transgenic seedlings after treatment with *E*. *coli;*
Fig. 9 shows images obtained in GUS analysis of transgenic seedlings after treatment with nanoparticles and corresponding metal ions (shoot);
Fig. 10 shows images obtained in GUS analysis of transgenic seedlings after treatment with nanoparticles and corresponding metal ions (root);
Fig. 11 shows a diagram illustrating the effects of nanoparticles and ions on GUS gene expression in HyPRO seedlings;
Fig. 12 shows a diagram illustrating relative expression of HyPRO seedlings following co-cultivation with *Agrobacterium tumefaciens* and *Escherichia coli;*
Fig. 13 shows a diagram illustrating relative expression of the GUS gene in HyPRO seedlings under various stress conditions;
Fig. 14 shows a diagram illustrating relative expression of the GUS gene in different plant parts of HyPRO transgenic plants;
Fig. 15 shows a nucleotide sequence SEQ ID NO: 2 of the HyPRO reverse primer designed to specifically amplify the promoter region using PCR;
Fig. 16 shows a nucleotide sequence SEQ ID NO: 3 of the HyPRO reverse primer designed to specifically amplify the promoter region using PCR.
Fig. 17 shows the expression of GUS gene under the control of HyPRO in prokaryotes such as *Escherichia coli* and *Agrobacterium tumefaciens.*

### Detailed description of the invention

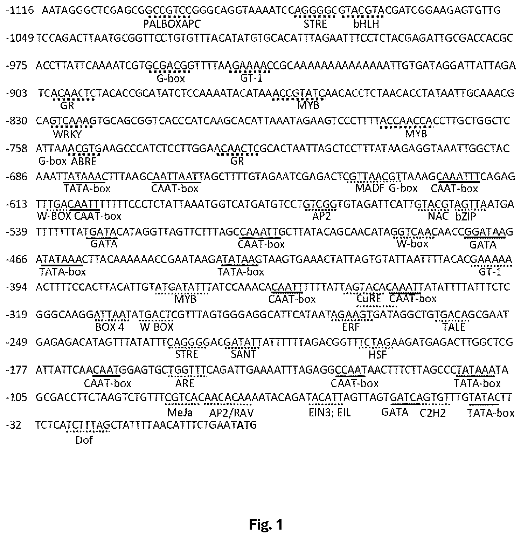

The nucleotide sequence of the promoter with cis-acting regulatory motifs according to the present invention is shown in fig. 1 (SEQ ID NO:1). The transcription start site (ATG) is shown in bold. HyPRO promoter sequence of 1116 base pairs. The fig. 1 shows TATA box: Located upstream of the transcription start site, this motif is essential for the binding of the transcription machinery. CAAT box: Another important regulatory element that enhances the efficiency of transcription initiation. Sequence of All the important boxes are underlined by solid lines and transcription factors and enhancers binding sites were underlined by dotted line.

The length of the HyPRO promoter sequence is 1116 base pairs. The promoter sequence was analyzed for cis-regulatory elements using online resources such as PlantCARE (https://bioinformatics.psb.ugent.be/webtools/plantcare/html/) and PlantPAN 2.0 (http://plantpan2.itps.ncku.edu.tw/index.html). The sequence similarity in the NCBI database is only 7%. PlantCARE is a comprehensive database that catalogues plant cis-acting regulatory elements, offering a portal for the in silico analysis of promoter sequences [Magali Lescot, Patrice Dhais, Gert Thijs, Kathleen Marchal, Yves Moreau, Yves Van de Peer, Pierre Rouz and Stephane Rombauts, Nucleic Acids Res. 2002 Jan 1;30(1):325-327]. It includes 435 different entries detailing transcription sites across more than 159 plant promoters. Similarly, PlantPAN 2.0 serves as a valuable resource for detecting transcription factor binding sites (TFBSs), the corresponding transcription factors (TFs), and other crucial regulatory elements like CpG islands and tandem repeats within a promoter or across a set of promoters in plants [Chi-Nga Chow, Han-Qin Zheng, Nai-Yun Wu, Chia-Hung Chien, Hsien-Da Huang, Tzong-Yi Lee, Yi-Fan Chiang-Hsieh, Ping-Fu Hou, Tien-Yi Yang, and Wen-Chi Chang "PlantPAN 2.0: an update of plant promoter analysis navigator for reconstructing transcriptional regulatory networks in plants", Nucleic Acids Res. 2015 : gkv1035v1-gkv1035]. The latest release of PlantPAN (version 2.0) encompasses 16,960 TFs and 1,143 TF binding site matrices across 76 plant species. The roles of identified regulatory motifs in the promoter were determined using these databases alongside relevant literature.

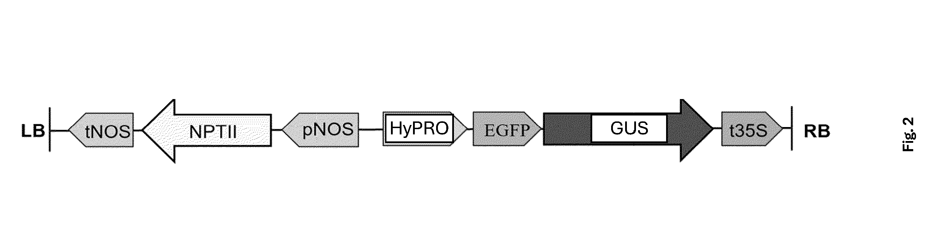

As mentioned above, fig. 2 shows a schematic map of the T-DNA region of the binary vector containing the GUS gene under the control of the HyPRO promoter. The construct includes the following elements from top to bottom: LB: Left Border sequence of the T-DNA region; tNOS: terminator sequence from the nopaline synthase gene, ensuring proper transcription termination; NPTII: Neomycin phosphotransferase II gene, providing kanamycin resistance for selecting transgenic plants; pNOS: promoter sequence from the nopaline synthase gene, driving the expression of the NPTII gene; HyPRO: HyPRO promoter sequence; EGFP: Enhanced Green Fluorescent Protein gene, used as a reporter gene to visualize expression; GUS: β-glucuronidase gene, also used as a reporter gene to monitor promoter activity; t35S: terminator sequence from the Cauliflower Mosaic Virus (CaMV) 35S gene, ensuring proper transcription termination; RB: Right Border sequence of the T-DNA region.

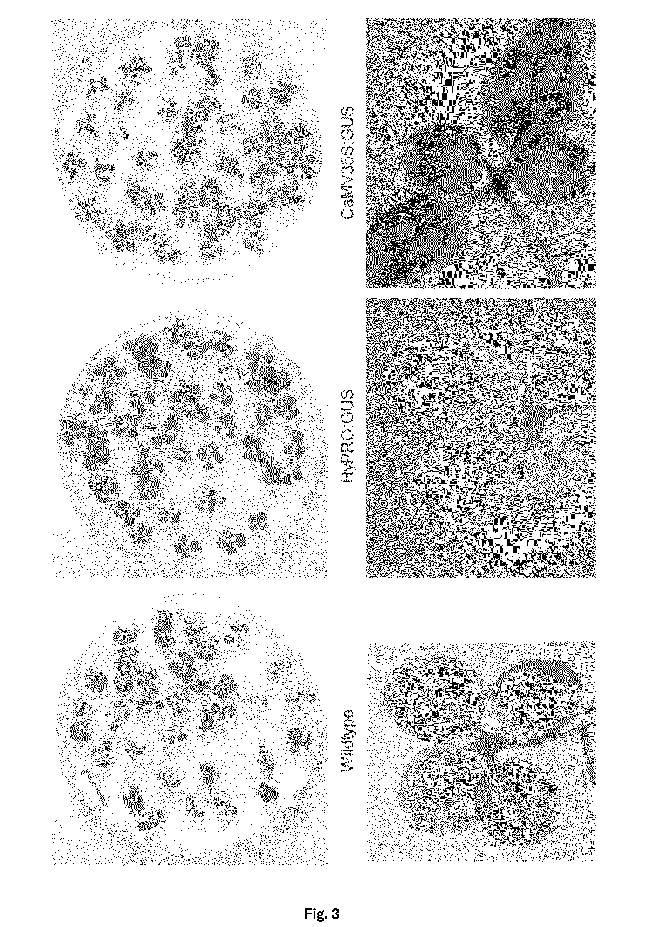

Further, as mentioned above, fig. 3 shows images of Petri dishes (upper row) and close-ups (lower row) of seedlings expressing the GUS gene under the control of HyPRO and CaMV35S show no change in phenotype compared to the wild type. The GUS assay of these seedlings shows constitutive expression of the GUS gene when driven by the CaMV35S promoter. The fig. 3 compares the expression of the GUS gene in wild-type plants, HyPRO transgenic plants, and CaMV35S transgenic plants. The images are divided into two rows: the upper row shows a Petri dish containing wild-type seedlings with no GUS expression, a Petri dish containing seedlings under the HyPRO promoter, and a Petri dish containing seedlings under the CaMV35S promoter; the lower row features a close-up of a wild-type seedling, a close-up of a seedling under the HyPRO promoter, and a close-up of a seedling under the CaMV35S promoter.

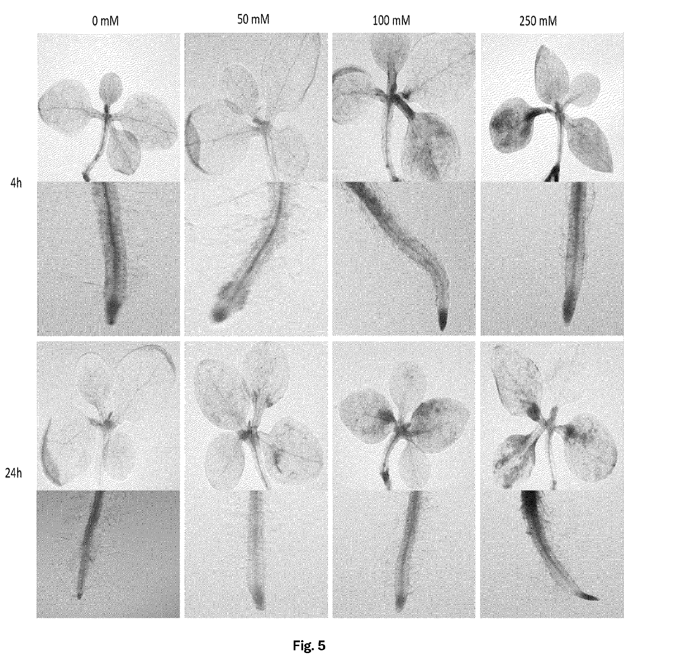

Fig. 5 shows images obtained in GUS analysis of transgenic seedlings after treatment with hydrogen peroxide (H₂O₂). More specifically, the fig. 5 shows the expression of the GUS gene in HyPRO seedlings under different concentrations of H₂O₂ over time. The seedlings were treated for 4 hours (top two rows) and 24 hours (bottom two rows) with various concentrations of H₂O₂: 0 mM, 50 mM, 100 mM, and 250 mM GUS expression was monitored in both the shoots and roots of the seedlings.

Isolating the promoter from *Hypericum perforatum,* a species that adapts to diverse environmental conditions, is expected to help in developing transgenic plants with increased resistance to stress factors such as drought, high and low temperatures, or pathogen attacks. The exemplary embodiment of the method for obtaining the inducible promoter according to the invention is presented in greater detail in Example 1 below.

Further, the exemplary embodiment of the method for producing a plant with modified gene expression according to the invention is presented in greater detail in Example 2 below.

Gene expression analysis: seedlings are exposed to nanoparticles for 24 hours, wrapped in aluminium foil to prevent contamination, and then immediately immersed in liquid nitrogen to quench biological processes and preserve RNA. If not processed immediately, samples are stored at -80°C to maintain RNA integrity. Total RNA is extracted from the collected seedlings using the InviTrap^{®} Spin Plant RNA Mini Kit (Invitek Molecular GmbH, Austria), ensuring high-quality RNA suitable for transcript analysis. The quality and quantity of the isolated RNA are assessed using spectrophotometry or gel electrophoresis to ensure purity and integrity before proceeding to cDNA synthesis. 1 µg of total RNA from each sample is reverse transcribed into first-strand cDNA using M-MLV Reverse Transcriptase (Promega, USA) following the manufacturer's instructions. This step is critical for converting RNA into a stable DNA format that can be used for PCR amplification. qRT-PCR is performed using the SensiFAST SYBR No-ROX Kit (Meridian Bioscience, USA) in a LightCycler 480 real-time PCR system (Roche, Basel, Switzerland). This system uses SYBR Green dye, which binds to double-stranded DNA, allowing quantification of gene expression as the PCR progresses. The expression levels of the GUS gene, driven by the HyPRO promoter, are quantified and normalized using the constitutive expression levels of housekeeping genes ACTIN and GAPDH. This normalization is crucial for accounting for any variability in cDNA amounts or sample loading errors. The method involves comparing the cycle threshold (Ct) values between the target gene and the reference genes, and between treated and control samples. The effect of nanoparticles on the induction of the HyPRO promoter is studied by comparing the gene expression in nanoparticle-exposed seedlings to controls. The GUS gene expression is analyzed both qualitatively through histochemical assays, which visually demonstrate enzyme activity, and quantitatively through qRT-PCR, providing a detailed measure of promoter activity under the influence of nanoparticles.

The present invention describes the HyPRO promoter, which is inducible and activates in response to both biotic and/or abiotic stresses. It is used in recombinant constructs, leading to increased gene expression when combined with target gene sequences and termination signals. The invention outlines methods for the isolation, characterization, and application of these promoters in genetic engineering, with a particular emphasis on breeding plants that are resistant to stress and promote growth, offering numerous benefits.

Controlling neoplastic diseases remains challenging through chemical and other conventional methods, as the physical presence of bacteria is not necessary for disease development once T-DNA is integrated into the plant genome. The quick induction of the promoter in response to Agrobacterium tumefaciens indicates that the promoter can recognize this phytopathogen. Therefore, the expression of antimicrobial genes under this promoter can help prevent neoplastic diseases caused by Agrobacterium in various plant species.

The strong induction of the new promoter under PEG treatment demonstrates its potential use in genetically engineering plants for drought tolerance. Additionally, the ability of the promoter to be induced by engineered nanoparticle treatment suggests that it can be used to detect the presence of these particles. For example, transgenic plants containing visible marker genes (for anthocyanin production) under the control of this promoter will change colour in the presence of nanoparticles.

Nanoparticles are increasingly used in various applications, including agriculture, and their interaction with plants is an important research area. The HyPRO promoter can help researchers track the presence of nanoparticles in plant tissues and assess their impact on gene expression and plant growth. By using the HyPRO promoter in plant genetic engineering and for detecting nanoparticles, researchers can gain valuable insights into gene expression patterns, genetic modifications, and the interactions of nanoparticles in plants. This information can ultimately contribute to the development of improved crop varieties and sustainable agricultural practices.

### Examples

### Example 1: Isolating the HyPRO promoter

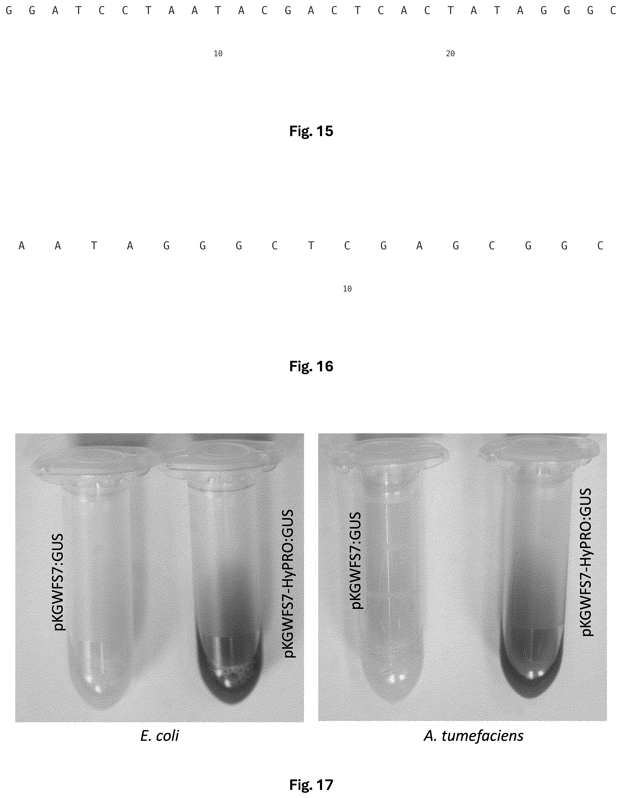

The method for obtaining the inducible HyPRO promoter according to the invention involves the following steps:
a) Isolating genomic DNA from leaves of *Hypericum perforatum*: leaves of *Hypericum perforatum* cultivars 'Helos' are harvested. Using the GenElute^{™} Plant Genomic DNA Miniprep Kit, genomic DNA is extracted following the manufacturer's protocol. Extracted DNA (2 µg) is digested with blunt cutting restriction enzymes FD Sspl and Dral to prepare for further cloning steps. The digested DNA is further purified using 3 M sodium acetate and ice-cold ethanol. Subsequent washing with 70% ethanol removes impurities, and the DNA is air-dried and re-dissolved in 20 µl sterile deionized water.
b) Identifying the promoter sequence: the phenolic oxidative coupling protein (Hyp-1) gene sequence is retrieved from NCBI (Accession number GU324244.1). Forward (SEQ ID NO: 2) fig. 15 and reverse (SEQ ID NO: 3) fig. 16 primers are designed to specifically amplify the promoter region using PCR.
(c) Cloning the identified promoter into suitable expression vectors using PCR cloning techniques: using the designed primers, a PCR is carried out to amplify the promoter region. Initially, random amplification of genomic ends (RAGE-PCR) is used with gene-specific primers and adaptors. The first PCR product is diluted and used as a template for a second, nested PCR to ensure specificity and increase yield.
(d) Ligation of the DNA into a cloning vector: preparation of vector and insert; the PCR product (1300 bp) is purified from a gel and ligated into the pJET1.2/blunt cloning vector using the CloneJet PCR Cloning Kit, following the manufacturer's recommendations. The ligation mixture is used to transform competent *Escherichia coli* DH5α cells.
(e) Selection of positive colonies: colony screening, transformed E. *coli* colonies are screened for the presence of the insert using colony PCR or restriction digest analysis. Plasmid extraction, positive colonies are grown in culture, and plasmids are extracted using a mini prep kit.
(f) Sequencing the cloned plasmids to confirm the correctness of the promoter sequence. The plasmids from positive colonies were sequenced. Sequencing results are analysed using Serial Cloner software to confirm the sequence and orientation of the cloned promoter.

The promoter sequence was amplified and cloned into the Gateway Cloning Entry Vector pGenDONR. The promoter was transferred by recombination from the entry vector (pGenDONR-HyPRO) into the destination binary vector pKGWFS7 (VIB, Ghent University, Ghent, Belgium) which has GUS gene downstream of recombination site. Recombination was performed with the Gateway^{™} LR Clonase^{™} II enzyme mix (Invitrogen, USA) according to the manufacturer's protocol to construct the binary vector pKGWFS7-HyPRO:GUS (Fig. 2). After recombination, the vector was transformed into competent E. *coli* (DH5α) cells using the freeze-thaw method and plated onto LB medium plates containing 100 mg/L spectinomycin (Sigma, USA). Positive colonies were identified using colony PCR and confirmed using sequencing. Further, the vector was transformed into *Agrobacterium tumefaciens* (EHA105) competent cells using freeze thaw method and plated onto LB medium plates containing 20 mg/L rifampicin and 100 mg/L spectinomycin. Positive colonies were further selected by colony PCR and reconfirmed by sequencing.

### Example 2: obtaining a Nicotiana tabacum plant with modified gene expression

The method for obtaining a *Nicotiana tabacum* plant with modified gene expression induced by HyPRO promoter according to the invention involves the following steps:
(a) Transforming a *Nicotiana tabacum* (tobacco) plant cell, tissue, organ, or embryo with a vector containing the recombinant cassette: tobacco leaves grown in vitro are cut into uniform discs using sterile tools and pre-cultured on MSA (Murashige and Skoog Agar) plates. Leaf discs are co-cultivated with *Agrobacterium tumefaciens* strain EHA105, which contains the binary vector pKGWFS7 with the GUS gene under the control of the HyPRO promoter. The culture is grown until it reaches an optical density (OD600) of 0.8-1.0. The cultured *Agrobacterium* is harvested at 4000 rpm for 15 minutes, and the pellet is resuspended in MS (Murashige and Skoog) basal medium to serve as the infection medium. The prepared leaf discs are immersed in the *Agrobacterium* suspension, then placed back onto the same MSA plates used for pre-culturing and incubated in the dark at 25°C for 2 days to allow for T-DNA transfer.
(b) Selecting the transformed cells, cell calli, embryos, or seeds: after co-cultivation, leaf discs are washed with Ticarcillin 2NA (500 mg/L) to remove excess *Agrobacterium,* reducing the risk of overgrowth in subsequent steps. The washed discs are transferred to MS agar plates supplemented with 1 mg/L BAP (Benzylaminopurine) and 0.1 mg/L NAA (Naphthaleneacetic acid) to promote shoot induction. These plates are incubated at 25°C, with media changes every 15 days, until the shoots are sufficiently developed.
(c) Regenerating plants from transformed cells, cell calli, embryos, or seeds: developed shoots are transferred to MS medium supplemented with 0.5 mg/L IBA (Indole-3-butyric acid) to encourage root development. Once rooted, the plants are moved to a greenhouse to undergo hardening, a process that acclimates the plants to less controlled, more natural conditions.
(d) Selecting mature plants from step (c) which have modified gene expression compared to non-transformed plants: mature plants are assessed for modified gene expression through histochemical GUS assays and quantitative real-time PCR (qRT-PCR). Plants showing successful integration and expression of the transgene are selected for further studies or breeding.

### Example 3: GUS transient assays comparing HyPRO and CaMV35S promoters in transgenic Nicotiana tabacum plants

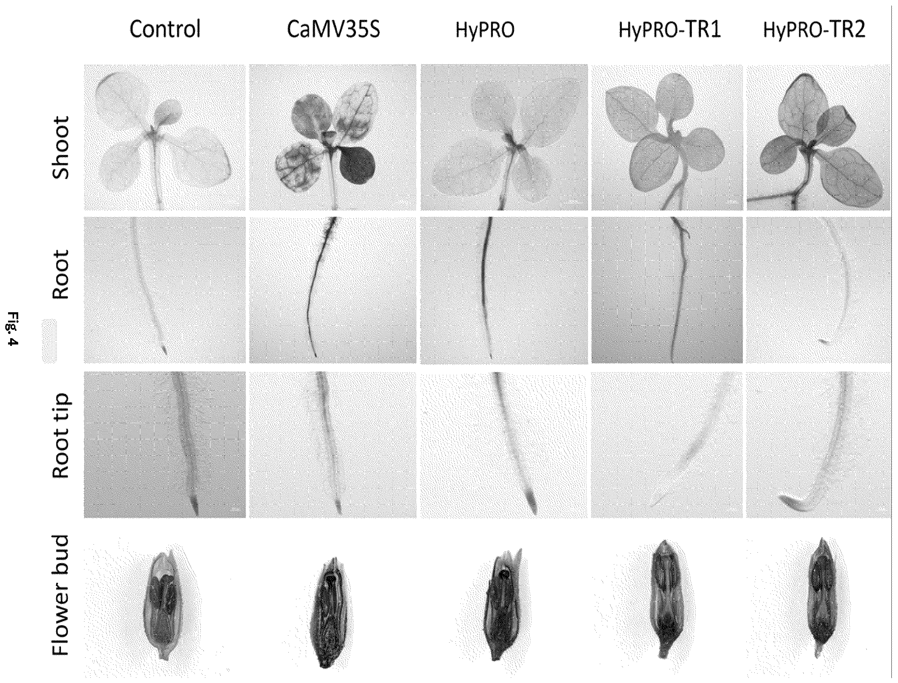

Fig. 4 shows images obtained in GUS transient assays in transgenic plants of *Nicotiana tabacum* having HyPRO full length, truncated and positive control having CaMV35S promoter linked to GUS. More specifically, fig. 4 shows comparative images of GUS expression in various parts of transgenic plants expressing different versions of the HyPRO promoter, as well as a positive control and a non-transgenic control. The analyzed plant parts include shoots, roots, root tips, and flower buds. The rows represent different constructs or controls, while the columns represent different plant parts: 'HyPRO' shows transgenic plants with the full-length HyPRO promoter; 'HyPRO-TR1' shows transgenic plants with the HyPRO-TR1 promoter (truncated version 1); 'HyPRO-TR2' shows transgenic plants with the HyPRO-TR2 promoter (truncated version 2); 'CaMV35S' serves as a positive control with transgenic plants expressing the CaMV35S promoter; 'Control' indicates non-transgenic plants (negative control).

The constitutive GUS activity was higher in the CaMV35S:GUS transgenic plants compared to HyPRO:GUS. GUS expression was found to be significantly reduced in the HyPRO-TR1 (728 bp) truncated constructs, whereas it was about to decline in the HpHyPRO TR2 (488 bp) truncated constructs. Low level of GUS activity has been detected in the HyPRO:GUS flowering bud. The results confirmed the significantly higher GUS constitutive expression in the presence of CaMV35S compared HyPRO:GUS in the transgenic tobacco leaves, flower parts and seedlings. However, the expression in root is higher in the case of HyPRO:GUS compared to CaMV35S transgenic seedlings.

### Example 4: Analysis of GUS gene expression in HyPRO seedlings after treatment with nanoparticles and ions

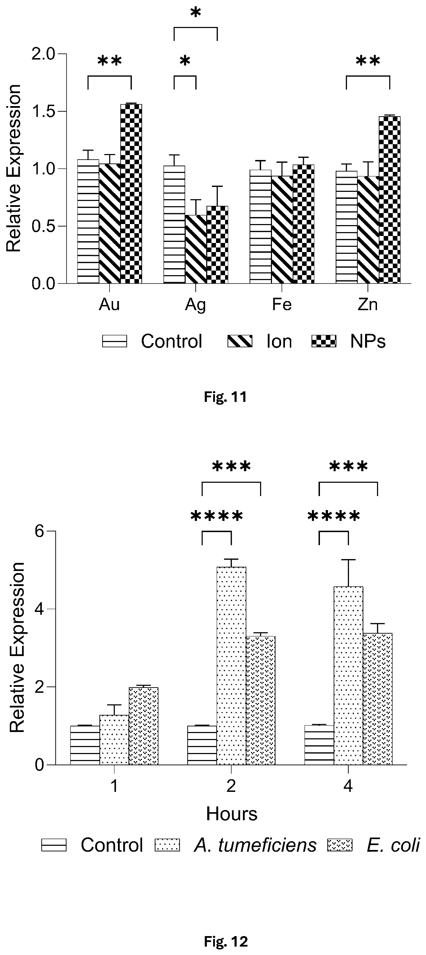

Relative expression of GUS gene in HyPRO:GUS seedlings treated with NPs. As shown in Fig. 11, Dunnett's test for multiple comparisons was performed for the significance test between treatments and between treatments and control. More specifically, fig. 11 shows the relative expression of the GUS gene in HyPRO seedlings after treatment with different types of nanoparticles (NPs) and their corresponding ions, in comparison to an untreated control. Measurements of GUS expression were taken for four different elements: gold (Au), silver (Ag), iron (Fe), and zinc (Zn).

The error bars indicate the standard error (SE). Briefly, two-week-old HyPRO:GUS seedlings were treated with 1 ppm of nanoparticles such as gold (AuNPs), silver (AgNPs), 50 ppm of iron oxide (Fe₂O₃), zinc oxide (ZnONPs) and the corresponding ions namely gold (AuCl₃), silver (AgNO₃), iron (FeSO₄) and zinc (ZnSO₄) ions for 24 hours. All treatments with nanoparticles and ions were performed in half MS medium at 22°C in a tissue culture laboratory with a photoperiod of 16 hours of light and 8 hours of darkness. Total RNA was isolated from the tissues and quantitative real-time PCR analysis was performed. Dunnett's test for multiple comparisons was performed for the significance test between the treatments and between the treatments and the control. Significance is indicated by one asterisk (p-value < 0.05); two asterisks (p-value < 0.01); three asterisks (p-value < 0.001), four asterisks (0.0001) at the top of the columns. The error bars indicate the standard error. Relative GUS expression was higher in the presence of AuNPs and ZnONPs, while lower GUS expression was observed in the presence of silver ions and AgNPs. Conversely, the presence of Au, Fe and Zn ions showed no significant difference in GUS expression compared to the control. Treatment with Fe₂O₃ NPs showed no difference in GUS expression compared to the control.

The results show that treatment with gold and zinc nanoparticles leads to significantly higher GUS expression compared to the control and ion treatments of these elements.

### Example 5: Relative expression of HyPRO:GUS seedlings after co-cultivation with Agrobacterium tumefaciens (strain EHA105) and E. coli (strain DH5α)

Two-week-old HyPRO:GUS seedlings were treated with either *Agrobacterium* or E. *coli* in half MS medium, while control seedlings received only half MS medium. All treatments were performed at 22°C in the tissue culture laboratory with a 16-hour light/8-hour dark photoperiod. Total RNA was isolated from the tissues and quantitative real-time PCR analysis was performed. As shown in Fig. 12, Dunnett's test for multiple comparisons was performed for the significance test between the treatments and between the treatments and the control. More specifically, fig. 12 shows the relative expression of the GUS gene in HyPRO seedlings after co-cultivation with *Agrobacterium tumefaciens* and *Escherichia coli* for a different time: 1 hour, 2 hours, and 4 hours. The graph compares the GUS gene expression in seedlings treated with *A. tumefaciens, E. coli,* and the control (untreated).

Significance is indicated by one asterisk (p-value < 0.05); two asterisks (p-value < 0.01); three asterisks (p-value < 0.001), four asterisks (0.0001) at the top of the columns. The error bars indicate the standard error. HyPRO-induced GUS expression was observed after 1, 2 and 4 hours of Agrobacterium and *E*. *coli* treatment in the HyPRO:GUS seedlings compared to the control. The HyPRO:GUS seedlings treated with *A. tumefaciens* and *E*. *coli* showed no significant differences in expression after one hour of treatment compared to the control. However, after two and four hours of treatments, a significant increase in GUS expression was observed in the HyPRO:GUS seedlings. Compared to the control, a 5-fold upregulation after 2 hours of and a 4-fold upregulation after 4 hours of *Agrobacterium treatment* was observed.

The highest GUS expression is observed after 2 hours and 4 hours of treatment with *A. tumefaciens,* indicating a strong response of the HyPRO promoter to this bacterial treatment. *E*. *coli* also induces GUS expression, but to a lesser extent than *A. tumefaciens.*

### Example 6: Relative expression of the GUS gene in HyPRO seedlings under various stress conditions

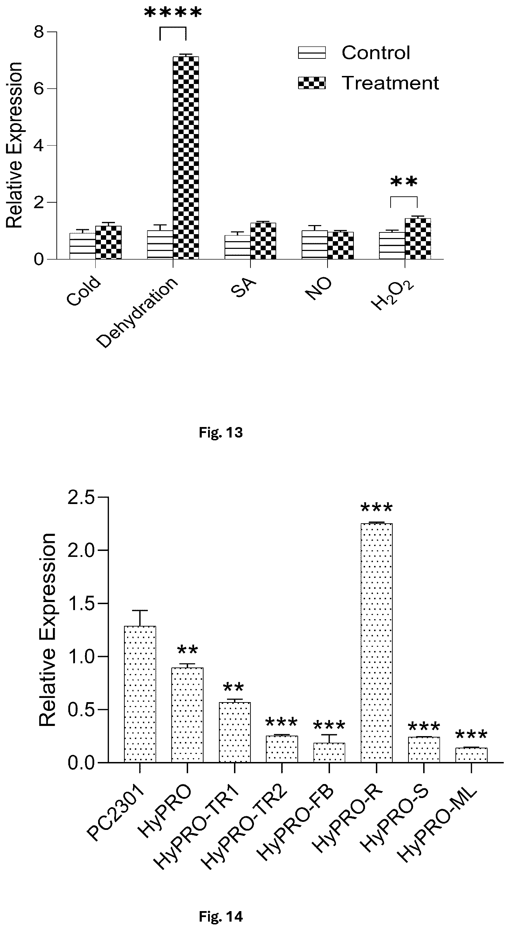

Relative expression of HyPRO:GUS seedlings treated with different stressors. Two-week-old seedlings of HyPRO:GUS seedlings were used for stress treatments. Dehydration treatment was performed using half MS with PEG 6000 (18% [w/v]) to stimulate desiccation stress for 24 h at 22°C in the tissue culture laboratory with a 16 h light/8 h dark photoperiod, while chilling treatment was performed at 4°C for 24 h. similarly, HyPRO:GUS seedlings were treated with 200 µM salicylic acid (SA), 200 µM sodium nitroprusside, a nitric oxide (NO) donor and 250 mM H₂O₂ for 24 h under similar conditions as mentioned above. All treatments were performed in half MS medium at 22°C in the tissue culture laboratory with a photoperiod of 16 h light/8 h dark. Total RNA was isolated from the tissues and quantitative real-time PCR analysis was performed. As shown in Fig. 13, Dunnett's test for multiple comparisons was performed for the significance test between the treatments and the control. More specifically, fig. 13 shows the relative expression of the GUS gene in HyPRO seedlings under various stress conditions. The bar graph compares GUS gene expression in treated seedlings with untreated control seedlings (control) across different stress treatments: cold (low temperature), dehydration, Salicylic Acid (SA), Nitric Oxide (NO), and Hydrogen Peroxide (H₂O₂).

Significance is indicated by one asterisk (p-value < 0.05); two asterisks (p-value < 0.01); three asterisks (p-value < 0.001), four asterisks (0.0001) at the top of the columns. The error bars indicate the standard error. A slight increase in GUS expression was observed with exogenous administration of 200 µM SA, whereas no differences were observed with cold and NO treatments. However, treatment with 18% PEG (polyethyleneglycols) induced GUS expression by 7.1-fold after 24 h of dehydration in HyPRO transgenic seedlings. H₂O₂ treatments increased GUS expression by 1.4-fold compared to the untreated control.

### Example 7: Relative expression of the GUS gene in different plant parts of HyPRO transgenic plants

Relative GUS genes expression in plant parts of HyPRO:GUS transgenic plants. Positive control PC2301 contains GUS gene drive by CaMV35S promoter and in all other case GUS gene drive by HyPRO. The HyPRO full length promoter (HyPRO), Truncated 1 (728 nucleotides) (HyPRO-TR1), Truncated 2 (HyPRO-TR2) (488 nucleotides); HyPRO-FB, GUS expression in HyPRO transgenic flower bud; HyPRO-R, GUS expression in HyPRO transgenic root; HyPRO-S, GUS expression in HyPRO transgenic stem, HyPRO-ML, GUS expression in HyPRO transgenic mature leaves (2 months old plants). Bar represents 200 µm. Dunnett's multiple comparison test was performed for the test of significance among the treatments and with the treatments and control. The label of significance is marked by one star (p-value < 0.05); two stars (p-value < 0.01); three stars (p-value < 0.001) on top of the columns. Error-bars denotes standard error.

The GUS expression was significantly lower in the leaves of HyPRO compared to the CaMV35S transgenic plants without induction. However, after PEG induction, the GUS reporter gene activity of HyPRO transgenic seedling was significantly enhanced while remaining same in CaMV35S transgenic seedling. Two 5' truncated HyPRO were obtained by deleting the upstream region of promoter and fusing them with GUS reporter gene like in full length promoter. The HyPRO Truncated construct 1 (HyPRO-TR1) is of 728 nucleotides and the HyPRO Truncated construct 2 (HyPRO-TR2) is of 488 nucleotides. The GUS activity was observed in the HyPRO-TR1 and HyPRO-TR2 compared to full length HyPRO. The visible small GUS activity was observed in the root of both HyPRO-TR1 and HyPRO-TR2. The GUS expression was reduced by 63% in HyPRO-TR1 truncated construct compared to the HyPRO. Furthermore, the GUS expression was reduced to 28% in the HyPRO-TR2 truncated construct compared to the HyPRO promoter (Fig. 14).

Fig. 14 shows a bar graph comparing the relative expression of the GUS gene in various HyPRO transgenic constructs and plant parts. From left to right, the graph includes: PC2301 (Positive Control), HyPRO (Full-length HyPRO promoter), HyPRO-TR1 (Truncated HyPRO promoter - 728 nucleotides), HyPRO-TR2 (Truncated HyPRO promoter - 488 nucleotides), and specific plant tissues including HyPRO-FB (GUS expression in flower buds), HyPRO-R (GUS expression in roots), HyPRO-S (GUS expression in stems), and HyPRO-ML (GUS expression in mature leaves from 2-month-old plants).

Comparing the HyPRO constitutive expression in various HyPRO transgenic plant parts, the GUS expression in seedlings, Flower buds, mature leaves, and stem was lower than the PC2301 transgenic seedling. However, higher constitutive GUS expression was observed in the root of HyPRO transgenic plants than the PC2301 (Fig. 14).

### Example 8: Expression of the GUS gene in HyPRO seedlings under different H₂O₂ concentrations over time

The fig. 4 shows the expression of the GUS gene in HyPRO seedlings under different concentrations of H₂O₂ (hydrogen peroxide) over time. The seedlings were treated for 4 hours (top two rows) and 24 hours (two bottom rows) with various H₂O₂ concentrations: 0 mM, 50 mM, 100 mM, and 250 mM GUS expression was monitored in both the shoots and roots of the seedlings.

**Table 1. Expression of the GUS gene in HyPRO seedlings under different H₂O₂ concentrations over time**

| **NaCl Concentration** | **Time (hours)** | **GUS Expression in Roots** | **GUS Expression in Shoots** |
|---|---|---|---|
| 0 mM (control) | 4 | Visible | None |
| 0 mM (control) | 24 | Visible | None |
| 50 mM | 4 | Visible | None |
| 50 mM | 24 | None | None |
| 100 mM | 4 | Visible | Visible |
| 100 mM | 24 | Visible | Moderate |
| 250 mM | 4 | Moderate | Visible |
| 250 mM | 24 | Moderate | Moderate |

The table 1 demonstrates that the expression of the GUS gene in HyPRO seedlings increases with higher concentrations of H₂O₂ and longer treatment durations. Notably, intense GUS expression is observed at higher H₂O₂ concentrations (100 mM and 250 mM) and after prolonged exposure (24 hours). According to the intensity, the GUS blue Colour stain in leaves and roots is divided into the several categories like None, visible, moderate, strong and intense.

### Example 9: Expression of the GUS gene in HyPRO seedlings under different stress conditions

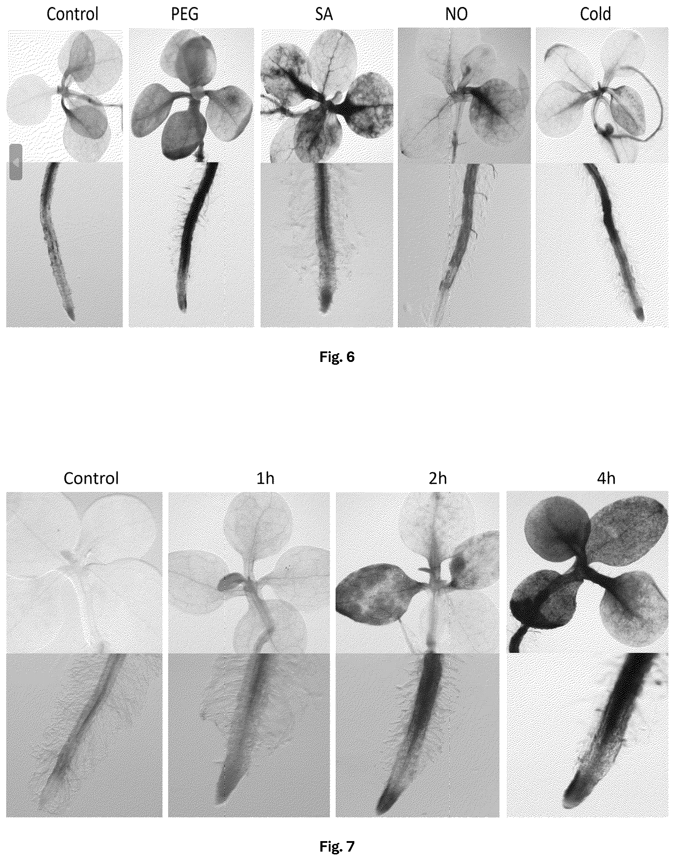

The fig. 6 shows the expression of the GUS gene in HyPRO seedlings subjected to different stress conditions, comparing the control (untreated) with other conditions. The panels depict seedlings treated with PEG (polyethylene glycol), SA (salicylic acid) NO (nitric oxide), and cold (low temperature). The images show both the shoots and roots of the seedlings.

**Table 2. Expression of the GUS gene in HyPRO seedlings under different stress conditions**

| **Treatment** | **Tissue** | **GUS Expression** |
|---|---|---|
| Control | Shoots | None |
| Control | Roots | Visible |
| PEG (polyethylene glycol) | Shoots | Strong |
| PEG (polyethylene glycol) | Roots | Intense |
| SA (salicylic acid) | Shoots | Moderate |
| SA (salicylic acid) | Roots | Moderate |
| NO (nitric oxide) | Shoots | Moderate |
| NO (nitric oxide) | Roots | Moderate |
| Cold (low temperature) | Shoots | Visible |
| Cold (low temperature) | Roots | Visible |

The table 2 demonstrates that GUS gene expression is induced under different stress conditions such as PEG, salicylic acid, nitric oxide, and cold, compared to the control. The highest GUS expression is observed under PEG and salicylic acid treatments, indicating a strong response of the HyPRO promoter to these stressors.

### Example 10: GUS analysis of transgenic seedlings after treatment with Agrobacterium tumefaciens

The fig. 7 shows the expression of the GUS gene in HyPRO seedlings subjected to infection by *Agrobacterium tumefaciens.* The seedlings were treated for 1 hour (first column), 2 hours (second column), and 3 hours (third column). The images show both the shoots and roots of the seedlings.

**Table 3. GUS analysis of transgenic seedlings after treatment with Agrobacterium tumefaciens**

| **Time (hours)** | **Tissue** | **GUS Expression** |
|---|---|---|
| Control | Shoots | None |
| Control | Roots | Visible |
| 1 | Shoots | None |
| 1 | Roots | Visible |
| 2 | Shoots | Visible |
| 2 | Roots | Strong |
| 3 | Shoots | Strong |
| 3 | Roots | Intense |

Table 3 demonstrates that GUS gene expression is induced under *Agrobacterium tumefaciens* treatment. The highest GUS expression is observed under the 3-hour treatment.

### Example 11: GUS analysis of transgenic seedlings after treatment with E. coli

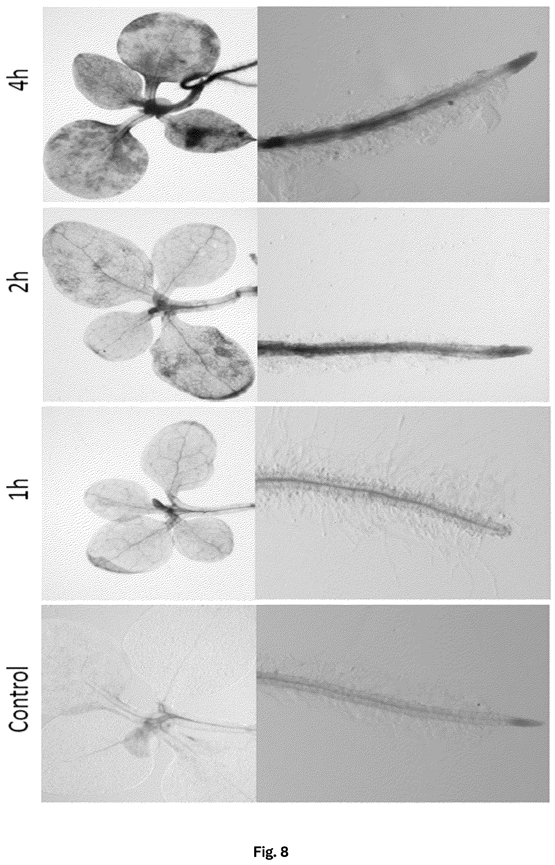

The fig. 8 shows the expression of the GUS gene in HyPRO seedlings subjected to E. *coli.* The seedlings were treated for 1 hour (first column), 2 hours (second column), and 3 hours (third column). The images show both the shoots and roots of the seedlings.

**Table 4. GUS analysis of transgenic seedlings after treatment with E. coli**

| **Time (hours)** | **Tissue** | **GUS Expression** |
|---|---|---|
| Control | Shoots | None |
| Control | Roots | None |
| 1 | Shoots | None |
| 1 | Roots | Visible |
| 2 | Shoots | Visible |
| 2 | Roots | Moderate |
| 3 | Shoots | Moderate |
| 3 | Roots | Strong |

Table 4 demonstrates that GUS gene expression is induced under E. *coli* treatment. The highest GUS expression is observed under the 3-hour treatment.

### Example 12: GUS analysis of transgenic seedlings after treatment with nanoparticles and corresponding ions (shoot and root)

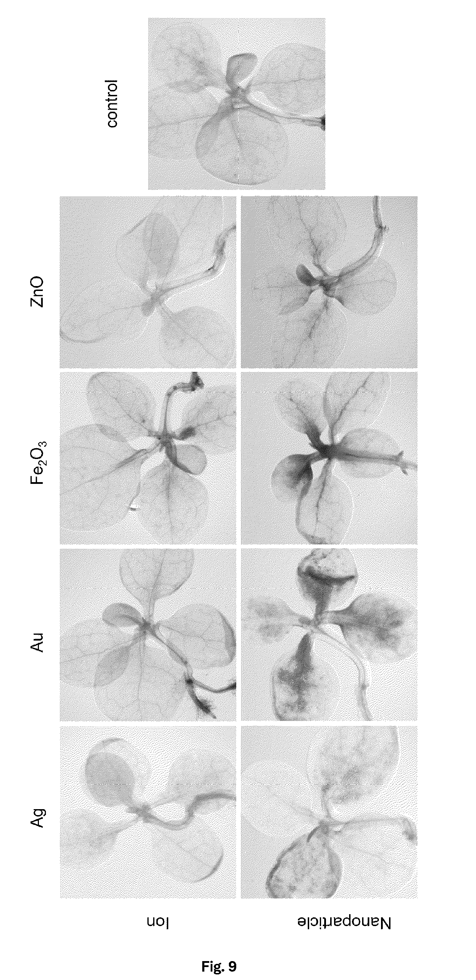

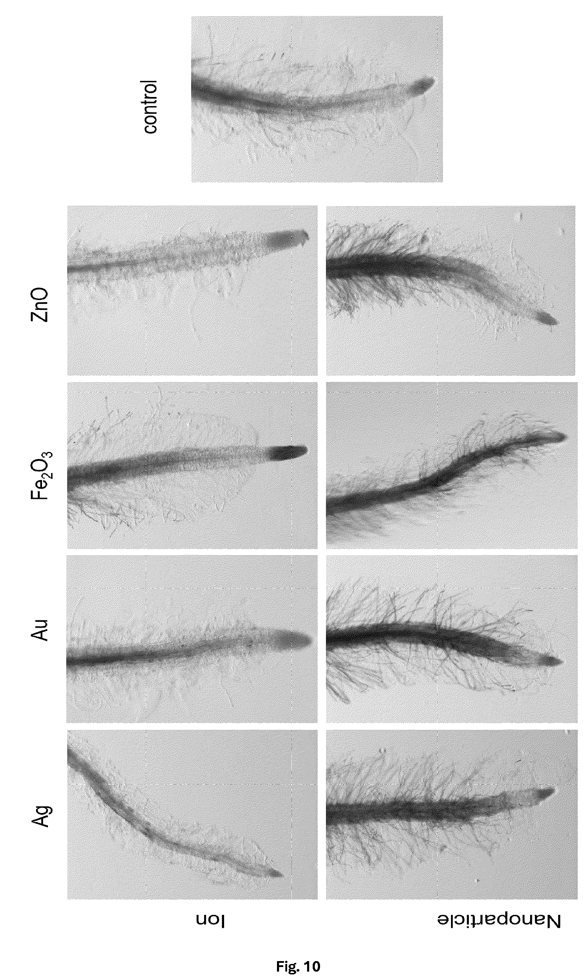

Fig. 9 and 10 show the expression of the GUS gene in HyPRO seedlings subjected to different nanoparticles (second row) and their corresponding ions (first row). Columns from left to right represent: silver (Ag), gold (Au), iron oxide (Fe₂O₃), zinc oxide (ZnO) and control. Fig. 9 shows the images of shoots, while fig. 10 shows the images of roots.

### Nanoparticles treatments:

The surface sterilized seeds of *Nicotiana tabacum* wild type and transgenic plants were germinated on half-strength MS (MS, Duchefa, Netherlands) agar (MSA) plates at 22 °C in a growth chamber with a 16 h light/8 h dark photoperiod. Two-weeks old six seedlings of HyPRO:GUS transgenic and wild type were used in the experiments. Gold (AuNPs; 1 ppm), Silver (AgNPs; 1 ppm), Iron oxide (Fe₂O₃; 50 ppm) and Zinc oxide (ZnO; 50 ppm) nanoparticles (US Research Nanomaterials, USA) were tested. The nanoparticles were sonicated for 10 min prior to treatment with seedlings. Seedlings were exposed to nanoparticles in half strength Murashige and Skoog liquid medium for 24 h at 22°C in a growth chamber with a 16 h light/8 h dark photoperiod. Seedlings treated with only medium considered as control.

**Table 5. GUS analysis of transgenic seedlings after treatment with nanoparticles and corresponding ions (shoot and root)**

| **Treatment** | **Nanoparticle/lon** | **GUS Expression in Shoots** | **GUS Expression in Roots** |
|---|---|---|---|
| Control | - | None | Visible |
| ZnO | Nanoparticle | Vsible | Visible |
| Fe₂O₃ | Nanoparticle | Visible | Strong |
| Au | Nanoparticle | Strong | Moderate |
| Ag | Nanoparticle | Strong | Visible |
| Control | Ion | None | Visible |
| ZnO | Ion | None | Visible |
| Fe₂O₃ | Ion | Moderate | None |
| Au | Ion | Moderate | None |
| Ag | Ion | None | Visible |

The table 5 presents the levels of GUS expression in the shoots and roots of plants under the influence of various nanoparticles and ions. Nanoparticles of ZnO and Fe₂O₃ induce moderate GUS expression in both shoots and roots. Nanoparticles of Au and Ag induce strong GUS expression in both tissues. This suggests that these nanoparticles have a stronger impact on the induction of GUS expression than ZnO and Fe₂O₃. Similarly to nanoparticles, ions of ZnO and Fe₂O₃ also induce moderate GUS expression in both tissues. Ions of Au and Ag also cause strong GUS expression in both tissues, suggesting that not only the nanoparticle form but also the ions of these metals have a strong impact on GUS expression

### Example 13: GUS gene expression under the control of HyPRO in prokaryotes

Fig. 17 shows the expression of HyPRO:GUS in prokaryotes such as *Escherichia coli* and *Agrobacterium tumefaciens.* In brief, a single colony of Escherichia coli and Agrobacterium tumefaciens containing the binary vector pKGWFS7-HyPRO:GUS was cultured in 5 ml of LB broth for 24 hours and centrifuged at 1240 x g (Eppendorf Centrifuge 5804, Rotor FA-45-6-30) for 10 minutes to pellet the bacteria. The bacterial pellet was carefully resuspended in a solution containing 0.5 mg/mlX-Gluc, 20 mM phosphate buffer (pH 7.0), 10% Triton, 10 mM EDTA, 5 mM K₃FeCN₆ and 5 mM K₄FeCN₆. After 12 hours, the solution was photographed with a DSLR camera.

## Claims

1. A promoter comprising a sequence selected from:
(a) a nucleic acid sequence defined in SEQ ID NO: 1, or
(b) a functional portion thereof, or
(c) a functional derivative or a homologous promoter which is at least 70% similar to one of them.

2. The promoter according to claim 1, wherein the promoter is inducible.

3. The promoter according to claim 2, which is activated in response to a biotic and/or abiotic stress.

4. The promoter according to claim 3, wherein the biotic stress includes any plant pathogen or beneficial microorganism.

5. The promoter according to claim 3, wherein the abiotic stress is caused by at least one stressor selected from of nanoparticles, excessive or insufficient light, high or low temperature, draught, hormones and chemicals.

6. The promoter according to one of claims 1-5, which is derived from the plant kingdom.

7. The promoter according to claim 6, wherein said sequence is derived from the Hypericaceae family.

8. The promoter according to claim 7, wherein said plant belonging to the genus Hypericum.

9. The promoter according to claim 8, wherein said sequence is derived from Hypericum perforatum.

10. A recombinant cassette comprising: (i) the nucleic acid sequence defined in SEQ ID NO: 1 operably linked to (ii) a polynucleotide sequence encoding a gene product to be transcribed from the inducible promoter, and (iii) a termination sequence.

11. A host plant cell transformed with a vector containing the recombinant cassette defined in claim 10.

12. A plant, its part, propagule or progeny thereof expressing the recombinant cassette defined in claim 10, or a transformed plant cell defined in claim 11.

13. The plant according to claim 12, which belongs to the Dicotyledons, and preferably is Nicotiana tabacum.

14. Use of the promoter defined in one of claims 1-9 for the enhanced expression of a nucleotide sequence in an organism.

15. The use according to claim 14, wherein the nucleotide sequence is a gene.

16. The use according to claim 14 or 15, wherein the organism is an eukaryote or prokaryote.

17. The use according to claim 16, wherein the eukaryote belongs to the plant kingdom.

18. The use according to claim 17, wherein the gene confers resistance of the plant to biotic and/or abiotic stress factors or promotes plant growth.

19. A method of introducing a vector containing the recombinant cassette of claim 10 for modifying gene expression in a plant, preferably Nicotiana tabacum.

20. A method for producing a plant with modified gene expression, comprising the following steps:
(a) transforming a plant cell, tissue, organ or embryo with a vector containing the recombinant cassette according to claim 10;
(b) selecting the transformed cells, cell calli, embryos or seeds;
(c) regenerating plants from transformed cells, cell calli, embryos or seeds selected in step (b);
(d) selecting mature plants from step (c) which have modified gene expression compared to non-transformed plants.

21. A method for obtaining an inducible promoter as defined in claim 3, comprising the following steps:
(a) isolating genomic DNA from leaves of *Hypericum perforatum*
(b) identifying the promoter sequence;
(c) cloning the identified promoter into suitable expression vectors using PCR cloning techniques;
(d) ligation of the DNA into a cloning vector
(e) selection of positive colonies
(f) sequencing the cloned plasmids to confirm the correctness of the promoter sequence.

22. The method according to claim 21, wherein the step (c) comprises PCR amplification using the forward primer having the nucleic acid sequence defined in SEQ ID NO: 2 and the reverse primer having the nucleic acid sequence defined in SEQ ID NO: 3 to obtain a PCR amplification product.
